# EUROPEAN PATENT SPECIFICATION

(11) **EP 2 833 934 B1**
(45) Date of publication and mention of the grant of the patent: **27.12.2017**
(21) Application number: 13718936.1
(22) Date of filing: 04.04.2013
(51) Int. Cl.: A61L 31/06, A61L 31/16, A61F 5/00

(54) **INTRAGASTRIC BALLOON SHELL MATERIALS AND CONSTRUCTION**
HÜLLMATERIALIEN FÜR INTRAGASTRISCHEN BALLON UND KONSTRUKTION
MATÉRIAUX D'ENVELOPPE DE BALLON INTRAGASTRIQUE ET SA CONSTRUCTION

(30) Priority: 05.04.2012 US 201213440831
(43) Date of publication of application: 11.02.2015
(60) Divisional application: 17201584.4
(73) Proprietor: APOLLO ENDOSURGERY, INC., Austin TX 78746 (US)
(72) Inventor: PAVLOVIC, Elizabeta, Santa Barbara, California 93105 (US); THOMPSON, Jordan M., Chino Hills, California 91709 (US); DANG, Loantrang, Ventura, California 93001 (US); GORALTCHOUK, Alexei, Rennsselaer, New York 12144 (US)
(74) Representative: Hoffmann Eitle
(86) International application number: PCT/US2013/035208
(87) International publication number: WO 2013/152154

(56) References cited:
- EP-A1- 1 671 590
- WO-A1-2011/097637
- US-A- 5 084 061
- US-A1- 2008 243 071
- US-A1- 2009 270 985
- EVZEN MACHYTKA ET AL: "Adjustable Intragastric Balloons: A 12-Month Pilot Trial in Endoscopic Weight Loss Management", OBESITY SURGERY ; THE JOURNAL OF METABOLIC SURGERY AND ALLIED CARE, SPRINGER-VERLAG, NEW YORK, vol. 21, no. 10, 8 May 2011 (2011-05-08), pages 1499-1507, XP019956185, ISSN: 1708-0428, DOI: 10.1007/S11695-011-0424-Z

## Description

### Field of the Invention

The present invention is directed to an intragastric device for the treatment of obesity and/or to facilitate weight loss and, in particular, to an inflatable intragastric balloon, such as an inflatable intragastric balloon endoscopically inserted into the stomach in an uninflated state, comprising particularly durable shell materials that enable implant periods of one year or more.

### Background of the Invention

Over the last 50 years, obesity has been increasing at an alarming rate and is now recognized by leading government health authorities, such as the Centers for Disease Control (CDC) and National Institutes of Health (NIH), as a disease. In the United States alone, obesity affects more than 60 million individuals and is considered the second leading cause of preventable death. In 2008, according to the World Health Organization (WHO), 1.5 billion adults, 20 and older, were overweight, and it is estimated that obesity affects at least 400 million adults.

Obesity is caused by a wide range of factors including genetics, metabolic disorders, physical and psychological issues, lifestyle, and poor nutrition. Millions of obese and overweight individuals first turn to diet, fitness and medication to lose weight; however, these efforts alone are often not enough to keep weight at a level that is optimal for good health. Surgery is another increasingly viable alternative for those with a Body Mass Index (BMI) of greater than 40. In fact, the number of bariatric surgeries in the United States is projected to reach approximately 400,000 annually in 2010.

Examples of surgical methods and devices used to treat obesity include the LAP-BAND® (Allergan Inc. of Irvine, CA) gastric band and the LAP-BAND AP® (Allergan). However, surgery might not be an option for every obese individual; for certain patients, non-surgical therapies or minimal-surgery options are more effective or appropriate.

Intragastric balloons are also known for treating obesity. One such inflatable intragastric balloon is described in U.S. Patent No. 5,084,061 and is commercially available as the Orbera® System from Allergan Inc. of Irvine, CA. These devices are designed to provide therapy for moderately obese individuals who need to shed pounds in preparation for surgery, or as part of a dietary or behavioral modification program.

The Orbera® System, for example, consists of a silicone elastomer intragastric balloon that is inserted into the stomach in an empty or deflated state and thereafter filled (fully or partially) with a suitable fluid. The balloon occupies space in the stomach, thereby leaving less room for food and creating a feeling of satiety for the patient. Placement of the intragastric balloon is non-surgical, trans-oral, usually requiring no more than 20-30 minutes. The procedure is performed gastroscopically in an outpatient setting, typically using local anesthesia and sedation. Intragastric balloons typically are implanted for a finite period of time, up to six months. Removing the balloon requires deflation by puncturing with a gastroscopic instrument, and either aspirating the contents of the balloon and removing it, or allowing the fluid to pass into the patient's stomach. Clinical results with these devices show that for many obese patients, the intragastric balloons significantly help to control appetite and accomplish weight loss.

The shell material of the Orbera® System balloon is a two-component high-temperature vulcanization (HTV) phenyl silicone with a platinum catalyzed curing system (trade name: PN-3206-04, 5% phenyl content) predispersed in Xylenes. Such a material has been proven suitable for implant durations of up to 6 months, but a material that permits implant for a year or longer would be desirable for improved therapeutic benefit and for sustained therapeutic benefit.

Therefore, despite many advances in the design of intragastric obesity treatment devices, there remains a need for improved devices that can be implanted for longer periods than before or otherwise address certain drawbacks of intragastric balloons and other such implants.

WO 2011/097637 A discloses a balloon of an intragastric device, comprising, in combination: a core of a first material and having an inner surface and an outer surface; and a coating of a second material on at least one of the inner surface of the core and the outer surface of the core. The first material may be of a more diemensional consistency than the second material, have a greater elasticity than the second material, or be primarily of polydimethylsiloxane.

### Summary of the Invention

The present invention addresses the above-described problems by providing a new intragastric balloon with at least a 9-month and preferably at least a 12-month implanted lifespan. The intragastric balloon has a shell made of a material with as good as or better than initial mechanical properties of previous materials; i.e. ultimate tensile strength (UTS) and elongation at break (Eb), lower stiffness (K), higher acid stability, and improved resistance to infection, with the latter two properties being particularly important. New materials with these properties should produce a device that has reduced adverse events and a longer lifespan *in vivo.*

In one embodiment, an intragastric balloon for the treatment of obesity, comprises (a) a shell having a wall enclosing an inner volume, and (b) a fluid fill valve secured in the shell wall, wherein a barrier layer of the shell wall is made of a 100% fluorinated room-temperature vulcanization (RTV) silicone. The 100% fluorinated room-temperature vulcanization (RTV) silicone may be CSM-3930. In one version, the shell wall includes an outer barrier layer of the 100% fluorinated room-temperature vulcanization (RTV) silicone and an inner scaffold layer of a different material. Desirably, the outer barrier layer is between about 5-25% of the total shell wall thickness, more preferably about 20% of the total shell wall thickness. The inner scaffold layer may be a 5% phenyl-substituted high-temperature vulcanization (HTV) silicone, or a methyl silicone, or a 100% fluorinated high-temperature vulcanization (HTV) silicone.

Another embodiment disclosed herein is an intragastric balloon for the treatment of obesity, comprising (a) a shell having a wall enclosing an inner volume, and (b) a fluid fill valve secured in the shell wall, wherein the shell wall, or a barrier layer thereof, is made of a 15-30% phenyl-substituted high-temperature vulcanization (HTV) silicone. The shell wall may be made of a 15% phenyl-substituted high-temperature vulcanization (HTV) silicone. In one embodiment, the shell wall includes an outer barrier layer of the 15-30% phenyl-substituted high-temperature vulcanization (HTV) silicone and an inner scaffold layer of a different material. Desirably, the outer barrier layer is between about 5-25% of the total shell wall thickness, more preferably about 20% of the total shell wall thickness. The inner scaffold layer may be a 5% phenyl-substituted high-temperature vulcanization (HTV) silicone, or a methyl silicone, or a 100% fluorinated high-temperature vulcanization (HTV) silicone.

Any of the intragastric balloons disclosed herein may further include a sealing layer in addition to the outer barrier layer and inner scaffold layer to provide a self-sealing shell in case of rupture. The intragastric balloons may also have an outer coating having an antimicrobial agent therein coated on the outer barrier layer.

A further understanding of the nature and advantages of the invention will become apparent by reference to the remaining portions of the specification and drawings.

### Brief Description of the Drawings

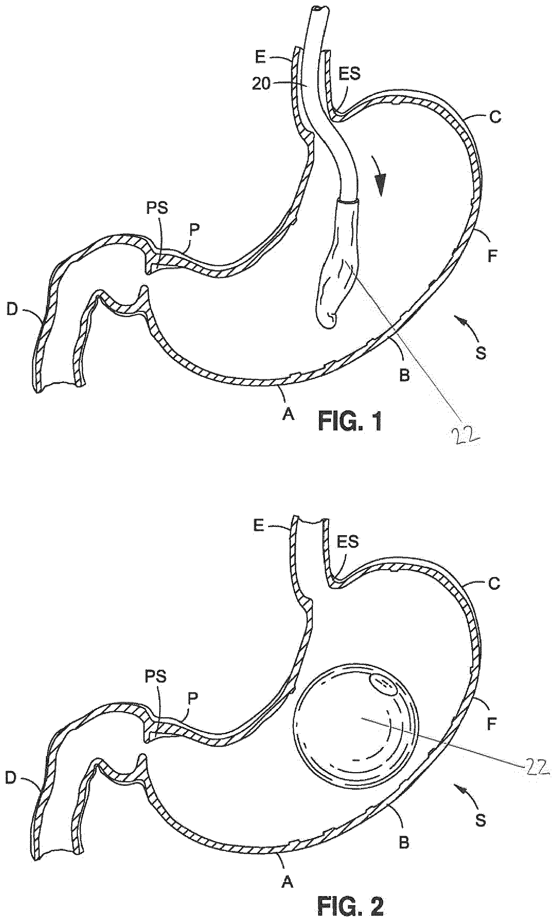

Features and advantages of the present invention will become appreciated as the same become better understood with reference to the specification, claims, and appended drawings wherein:
**[0001]** Figure 1 is a sectional view through a human stomach illustrating a collapsed/compressed intragastric obesity treatment implant prior to deployment within the stomach cavity and being expelled from the end of a flexible delivery member that is inserted down the esophagus;
**[0002]** Figure 2 illustrates the intragastric obesity treatment implant after deployment within the stomach cavity;
Figures 3-5 are graphs comparing the physical characteristics of a number of silicone-based materials after exposure to an acidic environment that simulates implant in the stomach cavity; and
Figures 6-8 are graphs comparing the physical characteristics of two silicone-based and a number of other materials after exposure to an acidic environment that simulates implant in the stomach cavity.

### Detailed Description of the Preferred Embodiments

The present invention is directed to intragastric devices for treating obesity by occupying space within the stomach and thereby limiting the available volume and slowing eating. Figure 1 illustrates the anatomy of the human stomach and the exemplary intragastric obesity treatment implant 22 therein. The major function of the stomach S is to temporarily store food and release it slowly into the duodenum D. The esophagus E extending downward from the mouth connects to the stomach via esophageal sphincter ES, which regulates flow food into the stomach cavity. The cardia C surrounds the superior opening of the stomach. The rounded portion superior to the body B and adjacent the cardia is the fundus F. Inferior to the fundus is the large central portion of the stomach, called the body that is lined with muscles that contract and relax repetitively to churn the food therein. The stomach processes the food to a semi-solid "chyme," which enables better contact with the mucous membrane of the intestines, thereby facilitating absorption of nutrients. In addition, the stomach is an important site of enzyme production.

Lower down in the stomach the antrum A connects the body to the pyloris P, which leads into the duodenum. Below the stomach, the duodenum D leads into the upper part of the small intestine (not shown). The region of the stomach that connects to the duodenum is the pylorus P. The pylorus P communicates with the duodenum D via the pyloric sphincter PS (valve). This valve regulates the passage of chyme from stomach to duodenum and it prevents backflow of chyme from duodenum to stomach.

While in the stomach cavity, the contents are subjected to a highly acidic environment. Gastric acid is the name given the digestive fluid produced by cells lining the stomach. It has a pH of 1 to 2 and is composed of hydrochloric acid (HCl) (around 0.5%, or 5000 parts per million), and large quantities of potassium chloride (KCl) and sodium chloride (NaCl). Gastric acid plays a key role in digestion of proteins, by activating digestive enzymes, and making ingested proteins unravel so that digestive enzymes can break down the long chains of amino acids. Other cells in the stomach produce bicarbonate to buffer the acid, ensuring the pH does not drop too low (acid reduces pH). The bicarbonate-secreting cells in the stomach also produce and secrete mucus. Mucus forms a viscous physical barrier to prevent gastric acid from damaging the stomach.

Figure 1 shows passage of a delivery tube 20 down the esophagus E and through the esophageal sphincter ES. An intragastric obesity treatment implant 22 comprises a balloon formed with an outer shell surrounding an inner cavity. During delivery, the implant 22 is deflated and elongated to pass through the delivery tube 20. After expulsion from the distal end of the delivery tube 20, saline is injected through a fill tube (not shown) connected to a fill valve 26 on one side of the implant 22. Once inflated, the fill tube is removed and retracted within the delivery tube 20, which is also removed up the esophagus and mouth. Figure 2 shows the implant 22 after deployment.

The shape of the implant 22 may be spherical as shown, or other shapes as desired. The implant shell has a capacity of approximately the same volume (400ml) as earlier intragastric balloons, which is proven to occupy a sufficient amount of space to facilitate weight loss.

The device is then left in place for a predetermined length of time, such as 6 months, or more preferably a year or more, while the patient is monitored for weight loss and any signs of malfunction. At the time of removal, a long overtube is placed down the esophagus E such that the tube ends reach from the mouth and into the stomach S. Next, a long-handled grasping tool or specially made removal device should be inserted down the overtube. By operating the grasper to grasp onto the side of the implant 22, the implant 22 can be pierced and deflated, and gently pulled, so as to retract it back into the overtube. The overtube is then retracted until it has been removed through the mouth. It is anticipated that this removal procedure will cause only slight throat irritation from the overtube, but will not induce excessive trauma.

The intragastric obesity treatment implant 22 is intended to be a single use implant placed in the stomach transorally without invasive surgery, and recovery time is believed to be minimal. The device may desirably be left in place one year or longer, which is somewhat material-dependent in the acidic stomach environment. The shell is formed of a material that will resist structural degradation over a period of at least twelve months within the stomach. In this context, the term "structural degradation" means that the device will retain structural integrity sufficiently to perform its intended function for a desired time period - in this case 12 months when filled to capacity or overfilled. Current balloons are typically filled to 400-700 mL, and 700 mL means overfilling. In one sense, the device is nonbiodegradable for the desired time period, though some biodegradation may commence short of structural degradation. Examples of possible materials follow.

Prior to a discussion of preferred new materials, a brief discussion of an exemplary material presently in commercial use is appropriate. Namely, the Orbera® System from Allergan Inc. of Irvine, CA utilizes a balloon having a shell made of a silicone elastomer material sold under the designation PN-3206-01 or PN-3206-04, which are two-component phenyl silicones by Nusil Silicone Technology of Carpinteria, CA. Both materials are in the family of materials sold by Nusil as MED-6400, which is a particular silicone with a platinum catalyzed curing system predisposed in Xylenes. Between 3206-01 and -04 there is a slight difference in viscosity (due a different solid content), and a slight difference in mechanical properties, which information can be found in the Nusil specification sheets for the 2 dispersions. Both PN-3206 silciones have a 5% phenyl content, and PN-3206-04, in particular, has proved suitable for use as the shell for a bariatric intragastric balloon for periods up to about six months.

Mechanical testing was performed on materials to assess their starting mechanical properties as well as their acid stability after they have undergone accelerated acid testing. The decrease in mechanical properties is a consequence of acid degradation on tested materials and consequently reflects the acid stability of these materials. In tests for new materials, the physical properties of PN-3206 will be treated as a baseline for comparison. Desirably, the candidate materials perform better in most if not all tests than PN-3206. In addition to accelerated testing in a relatively high acid concentration, additional "real-time" testing is desirable to provide confirmation or supplement the results. Real-time testing is preferably done using an actual intragastric balloon filled to its intended capacity and immersed in an acid solution that replicates the stomach environment. The balloon is left in the acid solution for long periods, conceivably as long as a year or more. Of course, such testing is by its nature time-consuming and more expensive, but provides added insight into the suitability of the various candidate materials.

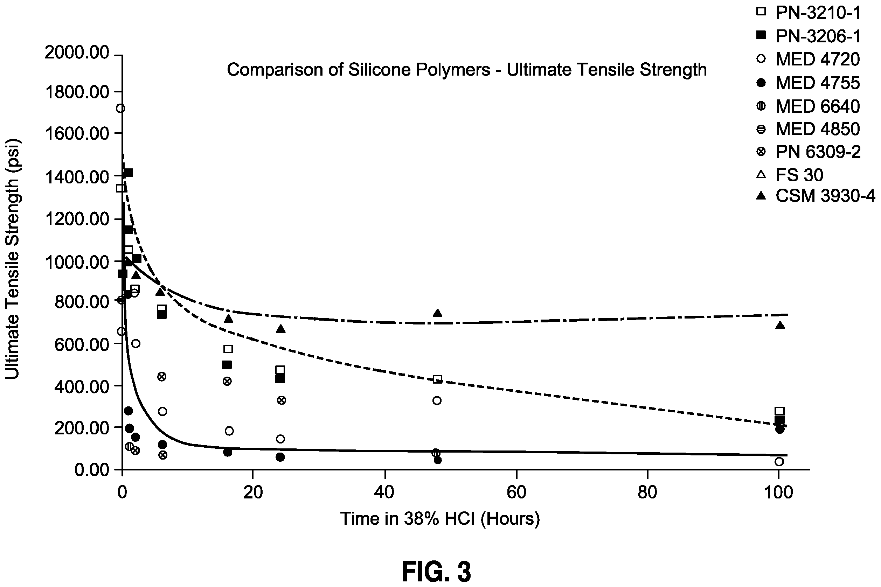

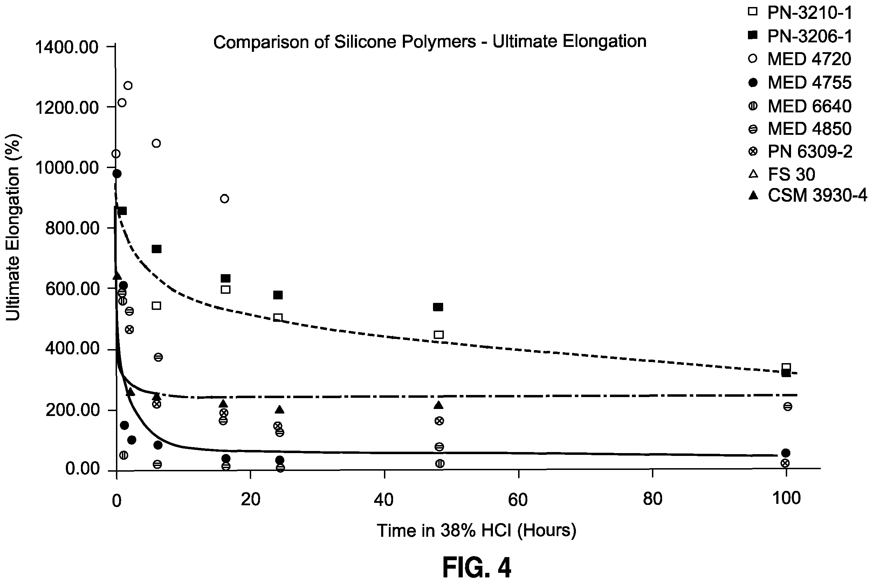

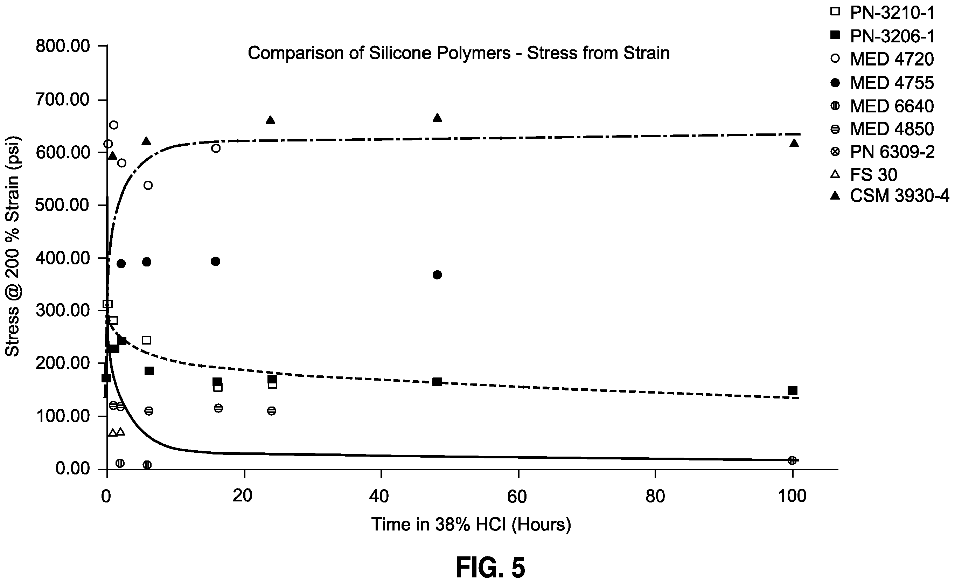

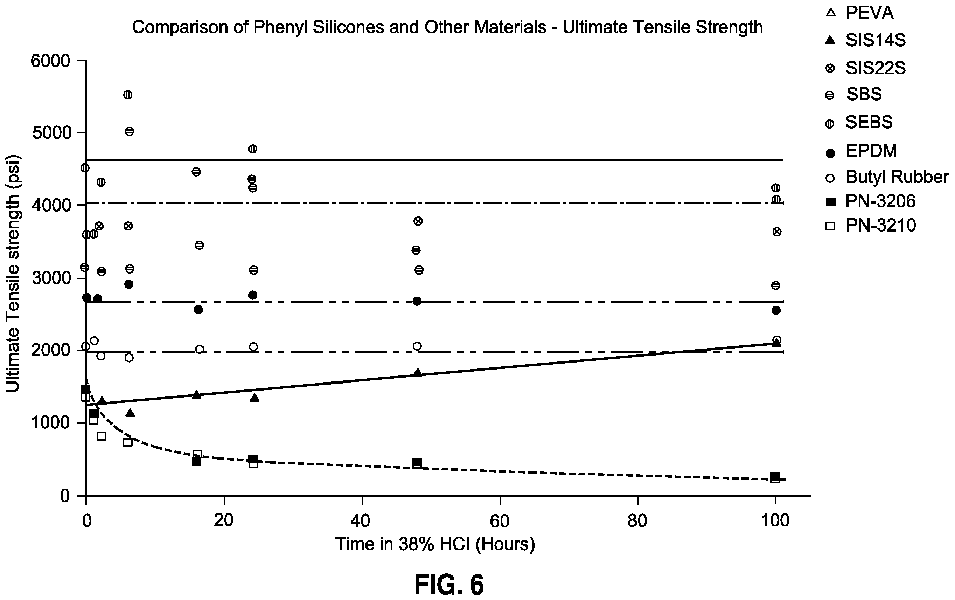

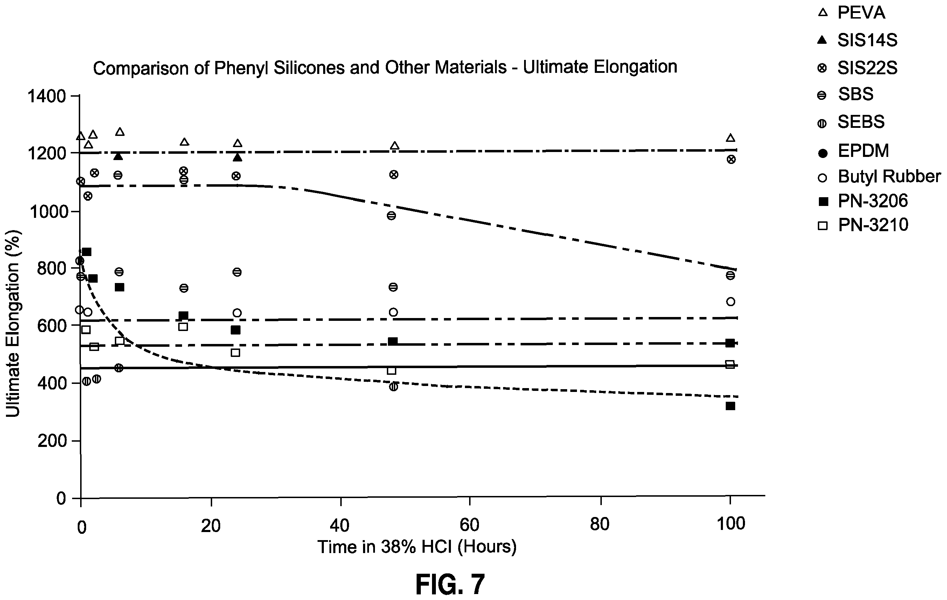

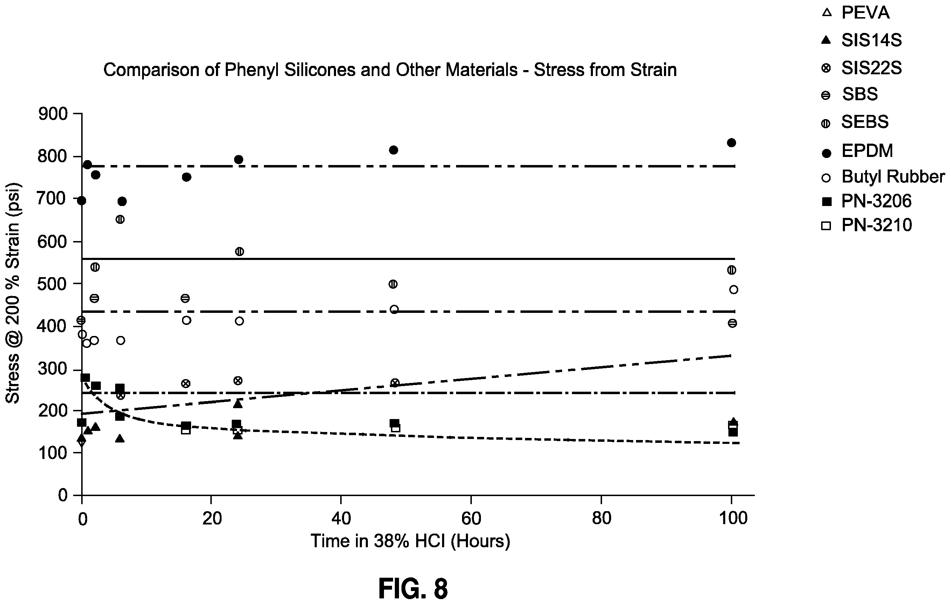

To investigate the suitability of new materials, the primary method is immersing the materials in a solution that simulates gastric acid. Prototypes are exposed to 0.1N hydrochloric acid (HC1) in vitro at body temperature (37° C.) for varying lengths of time, and then tested. The tables below and graphs of FIGS. 3-8 illustrate the physical characteristics of various materials after immersion in concentrated hydrochloric acid (HCl) for periods up to 100 hours (4 days, 4 hours). Future testing will subject promising candidates to the same conditions for periods up to 1, 3, 6 and 9 months, or until failure in order to mimic intragastric implantation conditions. The loss of mechanical properties of the materials are analyzed using mechanical testing. These tests are performed to determine real time acid stability and life expectancy of bariatric intragastric balloon prototypes manufactured using the current material (used as reference) and the new leading candidate materials.

As a baseline, the test results for the current Orbera® System shell material, PN-3206, are shown below in Table I:

**Table I - Performance of Current Material, PN-3206**

| | | | |
|---|---|---|---|
| Time (hr) Test → | Ultimate Tensile Strength, UTS (psi) | Elongation, Eb (%) | Stiffness, k (psi) |
| ↓ | | | |
| 0 | 1492(10.28MPa) | 827 | 230 (1.59 MPa) |
| 6 | 748(5.15MPa) | 737 | 189 (1.30 MPa) |
| 48 | 489(3.37MPa) | 537 | 169 (1.17 MPa) |
| 100 | 228(1.57MPa) | 313 | 148 (1.02 MPa) |

### Silicone Elastomers

One option for new shell materials is a different silicone elastomer. For instance, a material designated PN-3210-01 is a high temperature vulcanization (HTV) silicone (MED-6600 from Nusil Silicone Technology) dispersed in Xylene and with the same siloxane chain length as PN-3206, but with a higher phenyl content-15% instead of 5%. The higher phenyl content results in improved acid stability, as has been demonstrated by tests. Higher acid resistance is believed to be a significant factor in extending the in vivo life of the intragastric balloon shells. There is a significant statistical difference between PN-3206 (current material) and PN-3210 (new material, higher phenyl content), with the latter having better mechanical property retention. In general, a material that is 15-30% phenyl-substituted, but no higher, like PN-3210, is believed to be a good candidate for long-lasting intragastric balloons.

The same acid-exposure test results for PN-3210 are summarized below in Table II. In these results, comparison with the baseline results for PN-3206 are made so as to more easily determine the relative merit of the material. Good results are defined by values of UTS and Eb that are higher than or equal to baseline values from PN-3206 testing, and values of stiffness (k) that are lower than or equal to baseline values. Worse results are defined by values of UTS and Eb that are lower than baseline values, and values of stiffness (k) that are higher compared to baseline values. Ideally, the new material will demonstrate better than or equal to properties than the baseline material, and any such values are shown in bold.

**Table II - Performance of New Silicone Material, PN-3210**

| Time (hr) Test → | Ultimate Tensile Strength, UTS (psi) | Elongation, Eb (%) | Stiffness, k (psi) |
|---|---|---|---|
| ↓ | | | |
| 0 | 1341(9.24MPa) | 605 | 230 (1.59 MPa) |
| 6 | **768**(5.29MPa) | 546 | 247 (1.70 MPa) |
| 48 | 436(3.00MPa) | 444 | 166 (1.14 MPa) |
| 100 | **275**(1.89MPa) | **337** | 158 (1.09 MPa) |

It should be noted that in limited real-time testing using an actual intragastric balloon filled to its intended capacity and immersed in an acid solution that replicates the stomach environment, PN-3210 unexpectedly performed better than it did in the accelerated testing. Therefore, PN-3210 is considered a good candidate for a more durable intragastric balloon shell, either as the entire shell or as a barrier layer such as with a scaffold of PN-3206.

A number of new materials testing herein are believed to be good candidates for use in long-lasting intragastric balloons. Some of the materials have suitable material properties so as to be available for use as the entire shell wall, while other may only be suitable as a barrier layer. For instance, the materials underwent creep testing to determine their propensity to maintain a shape or otherwise to creep or change shape over time. Poor creep resistance means that a material will not maintain its balloon shape regardless of its ability to withstand the acidic stomach environment, and will be susceptible to permanent deformation and fold-induced crack formation. However, those materials may still be used as a "barrier layer" combined with a "scaffold layer" of a different material that does resist creep. For instance, the presently used material PN-3206 may be used as an underlying scaffold layer with one of the new materials layered on top as an outer barrier layer. Preferably, the barrier layer will be 5-25% of the shell thickness, and more preferably 20%. The barrier layer is preferably on the outside, although it could be within the shell.

### Fluorinated silicones

Another possible class of new materials is fluorinated silicones. For example, CSM-3930, a 100% fluorinated room-temperature vulcanization (RTV) silicone (MED-6655 from Nusil Silicone Technology) dispersed in Xylene, displays a better acid stability than PN-3206, although its starting mechanical properties are less advantageous. While the phenyl substituted silicones showed a continual decrease in mechanical properties over time in 12N HCl acid, the 100% fluorinated silicone only lost mechanical properties early on and then remained the same. This indicates that while some of the bonds in the fluorinated silicone are cleaved by the acid, the bulk of the material remains unaffected. The CSM-3930 silicone is also stiffer than the PN-3206 which would allow the shell to be thinner while still being able to hold the same break force. This is an advantage because doctors have indicated that a thinner shell would make implantation and explantation easier. CSM-3930 also should theoretically be more resistant to microbial infection due to it being 100% fluorinated.

A 100% fluorine substitution runs counter to the success of relatively low percentage phenyl-substituted compounds. For instance, as mentioned above, PN-3206-04, has a 5% phenyl content, and has proved suitable for use as the shell for a bariatric intragastric balloon for periods up to about six months. Moreover, progressive testing of fluoro-substitutions reveals that materials up to 50% percent fluorine substitution are not suitable. Therefore, one would expect the trend to continue, but instead a 100% fluorine substitution works well.

The acid-exposure test results for CSM-3930 are summarized below in Table III. The Table is set up the same as for all the other new materials. Once more, desirable new materials will demonstrate better results better than the baseline (PN-3206), which values are indicated in bold.

**Table III - Performance of New Fluorinated Silicone Material, CSM-3930**

| Time (hr) Test → | Ultimate Tensile Strength, UTS (psi) | Elongation, Eb (%) | Stiffness, k (psi) |
|---|---|---|---|
| ↓ | | | |
| 0 | 1156(7.97MPa)) | 476 | 371 (2.56 MPa) |
| 6 | **1056**(7.28MPa) | 368 | 499 (3.44 MPa) |
| 48 | **805**(5.55MPa) | 319 | 468 (3.23 MPa) |
| 100 | **978**(6.74MPa) | 317 | 507 (3.50 MPa) |

In limited real-time testing, CSM-3930 performed similar to the accelerated testing, and is therefore considered a good candidate for a more durable intragastric balloon shell. However, because of the presence of creep in CSM-3930, it is preferably only used as a barrier layer such as with a scaffold of PN-3206, or other suitable material.

Early stages of prototype building using CSM-3930 also indicated that a HTV version of the same material would be desirable. For this purpose, LSR-9744, a HTV 100% fluorinated silicone, was developed and is currently being tested for acid stability. The 100% fluorinated HTV silicone will have better starting mechanical properties than the RTV version making it a more suitable candidate. Although the LSR-9744 has better mechanical properties in general, tear strength is extremely low based on observations.

The acid-exposure test results for LSR-9744 are summarized below in Table IV. The Table is set up the same as for all the other potential materials. Once more, desirable new materials will demonstrate better results than the baseline (PN-3206), which values are indicated in bold.

**Table IV - Performance of New HTV Fluorinated Silicone Material, LSR-9744**

| Time (hr) Test → | Ultimate Tensile Strength, UTS (psi) | Elongation, Eb (%) | Stiffness, k (psi) |
|---|---|---|---|
| ↓ | | | |
| 0 | 831(5.72MPa) | 322 | 341 (2.35 MPa) |
| 6 | **1173**(8.08MPa) | 277 | 750 (5.17 MPa) |
| 48 | **1089**(7.50MPa) | 210 | 1004 (6.92 MPa) |
| 100 | **877**(6.04MPa) | 218 | 794 (5.47 MPa) |

Another fluorinated silicone (FS) tested is FS 30, where the "30" indicates fluorine content. FS 30 (ASC part #40130) was purchased from Applied Silicone Corporation (Santa Paula, CA).

The acid-exposure test results for FS 30 are summarized below in Table V. The Table is set up is the same as for all the other new materials, with values better than the baseline material (PN-3206) indicated in bold.

**Table V - Performance of New Fluorinated Silicone Material, FS 30**

| Time (hr) | Ultimate Tensile Strength, UTS (psi) | Elongation, Eb (%) | Stiffness, k (psi) |
|---|---|---|---|
| Test → | | | |
| ↓ | | | |
| 0 | **1423**(9.81MPa) | **917** | 166 (1.59 MPa) |
| 6 | 84(0.57MPa) | 31 | N/A |
| 48 | 58(0.39MPa) | 9 | N/A |
| 100 | N/A | N/A | N/A |

### Methyl Silicones

A number of so-called methyl silicones were also tested. This class of materials includes methyl, ethyl or CxHy silicones, such as MED-4720, CSM-4755, MED-6640, MED-4850, PN-6309. Such materials are not encompassed by the material requirements of the independent claims. None of these materials tested very well, and thus tabular results will be omitted. However, it is possible that a scaffold layer using methyl silicone material may be combined with a barrier layer of a different material that presents a better candidate for use in the durable intragastric balloons herein.

### Thermoplastic Elastomers (TPE)-Styrene Rubbers

Such materials are not encompassed by the material requirements of the independent claims.

Styrene-Isoprene-Styrene 14% Styrene (SIS14S) performs very well in all investigated mechanical properties and displays high acid stability. Nevertheless, this study did not include creep analysis and chemical resistance to oil and gastric enzymes. Also, the self-sealing ability of SIS14S and other thermoplastic elastomers when heated can complicate the manufacturing process.

The acid-exposure test results for SIS14S are summarized below in Table VI. The Table is set up is the same as for all the other new materials, with values better than the baseline material indicated in bold.

**Table VI - Performance of New TPE-Styrene Material, SIS14S**

| Time (hr) | Ultimate Tensile Strength, UTS (psi) | Elongation, Eb (%) | Stiffness, k (psi) |
|---|---|---|---|
| Test → | | | |
| ↓ | | | |
| 0 | **1496**(10.31MPa) | **1196** | **116 (0.79 MPa)** |
| 6 | **2324**(16.02MPa) | **1101** | **154 (1.06 MPa)** |
| 48 | **2778**(19.15MPa) | **1118** | **161 (1.11 MPa)** |
| 100 | **1811**(12.48MPa) | **1104** | **152 (1.05 MPa)** |

Styrene-Isoprene-Styrene, 22% Styrene (SIS22S) is stable in acid but displays a stiffness higher than the baseline. This polymers has a higher styrene content compared to the SIS14S, which explains the higher stiffness.

The acid-exposure test results for SIS22S are summarized below in Table VII. The Table is set up is the same as for all the other new materials, with values better than the baseline material indicated in **bold.**

**Table VII - Performance of New TPE-Styrene Material, SIS22S**

| Time (hr) | Ultimate Tensile Strength, UTS (psi) | Elongation, Eb (%) | Stiffness, k (psi) |
|---|---|---|---|
| Test → | | | |
| ↓ | | | |
| 0 | **3589**(24.74MPa) | **1108** | 261 (1.80 MPa) |
| 6 | **3752**(25.86MPa) | **1197** | 235 (1.62 MPa) |
| 48 | **4052**(27.93MPa) | **1130** | 267 (1.84 MPa) |
| 100 | **4120**(28.40MPa) | **1172** | 275 (1.90 MPa) |

Styrene-Butadiene-Styrene 30% Styrene (SBS) and Styrene-(Ethylene/Butylene)-Styrene 28% Styrene (SEBS) are stable in acid but display a stiffness higher than the baseline, and SEBS also has a lower elongation at break. These polymers have a higher styrene content compared to the SIS14S, which explains the higher stiffness. Therefore, for SBS and SEBS, selecting a copolymer with a lower styrene content may be the first step to improve mechanical response.

The acid-exposure test results for SBS and SEBS are summarized below in Tables VIII and IX, respectively. The Tables are set up is the same as for all the other new materials, with values better than the baseline material indicated in **bold.**

**Table VIII - Performance of New TPE-Styrene Material, SBS**

| Time (hr) | Ultimate Tensile Strength, UTS (psi) | Elongation, Eb (%) | Stiffness, k (psi) |
|---|---|---|---|
| Test → | | | |
| ↓ | | | |
| 0 | **3793**(26.15MPa) | **773** | 414 (2.85 MPa) |
| 6 | **5016**(34.58MPa) | **792** | 434 (2.99 MPa) |
| 48 | **3405**(23.47MPa) | **731** | 441 (3.04 MPa) |
| 100 | **3695**(25.47MPa) | **770** | 408 (2.81 MPa) |

**Table IX - Performance of New TPE-Styrene Material, SEBS**

| Time (hr) | Ultimate Tensile Strength, UTS (psi) | Elongation, Eb (%) | Stiffness, k (psi) |
|---|---|---|---|
| Test → | | | |
| ↓ | | | |
| 0 | **4543**(31.32MPa) | 455 | 551 (3.79 MPa) |
| 6 | **5537**(38.17MPa) | 455 | 655 (4.52 MPa) |
| 48 | **2264**(15.60MPa) | 424 | 518 (3.57 MPa) |
| 100 | **4259**(29.36MPa) | **458** | 530 (3.65 MPa) |

### Thermoplastic Elastomers (TPE)-Synthetic Rubbers

Such materials are not encompassed by the material requirements of the independent claims.

Polyethylene Vinyl Acetate (PEVA or Elvax) has good mechanical properties but its starting UTS is slightly lower than the baseline. The acid test shows the elongation of PEVA drops at 100 hours and its stiffness is increasing. Attenuated Total Reflectance-Fourier Transform Infrared (ATR-FTIR) spectroscopy of the acid-exposed samples (results not shown) indicates that the acetate is cleaved, generating acetic acid, and an OH is created on the vinyl acetate backbone. The increasing amount of OH inside the polymer can be responsible for the formation of H bonds, which in turn can cause the drop in elongation at break and increase in stiffness. It seems also to be an increase in UTS. In conclusion, the PEVA is affected by acid but the effect is mitigated.

The acid-exposure test results for Elvax are summarized below in Table X. The Table is set up is the same as for all the other new materials, with values better than the baseline material indicated in **bold.**

**Table X - Performance of New TPE-Synthetic Material, Elvax**

| | | | |
|---|---|---|---|
| Time (hr) | Ultimate Tensile Strength, UTS (psi) | Elongation, Eb (%) | Stiffness, k (psi) |

| Test → | | | |
|---|---|---|---|
| ↓ | | | |
| 0 | 1131(7.7 9 MPa) | **1077** | **213** (1.47 MPa) |
| 6 | **1147**(7.90MPa) | **1133** | **200** (1.38 MPa) |
| 48 | **1715**(11.82MPa) | **981** | 259 (1.79 MPa) |
| 100 | **2177**(15.00MPa) | **785** | 346 (2.39 MPa) |

Ethylene Propylene Diene Monomer (EPDM) and butyl rubber (BR). The acid stability of these rubbers is excellent but they have a poor mechanical response. This is in part due to the carbon black filler they contain. It can be anticipated that suppressing carbon black will decrease stiffness and increase elongation at break, but also that it will decrease UTS and increase creep. It can possibly affect their acid stability as well.

The acid-exposure test results for EPDM are summarized below in Table XI. The Table is set up is the same as for all the other new materials, with values better than the baseline material indicated in **bold.**

**Table XI - Performance of New TPE-Sythetic Material, EPDM**

| | | | |
|---|---|---|---|
| Time (hr) | Ultimate Tensile Strength, UTS (psi) | Elongation, Eb (%) | Stiffness, k (psi) |

| Test → | | | |
|---|---|---|---|
| ↓ | | | |
| 0 | **2751**(18.96MPa) | 576 | 704 (4.85 MPa) |
| 6 | **2940**(20.27MPa) | 576 | 600 (4.14 MPa) |
| 48 | **2726**(18.79MPa) | **562** | 797 (5.50 MPa) |
| 100 | **2568**(17.70MPa) | **531** | 731 (5.04 MPa) |

Two other synthetic TPEs tested were Butyl Rubber (BR) and Viton®. Viton® is a rubber that is a copolymer of hexafluoropropylene (HFP) and vinylidene fluoride (VDF), which is not and will not be approved for use in implantable devices.

The acid-exposure test results for BR are summarized below in Table XII. The Table is set up is the same as for all the other new materials, with values better than the baseline material indicated in **bold.**

**Table XII - Performance of New TPE-Synthetic Material, Butyl Rubber (BR)**

| Time (hr) | Ultimate Tensile Strength, UTS (psi) | Elongation, Eb (%) | Stiffness, k (psi) |
|---|---|---|---|
| Test → | | | |
| ↓ | | | |
| 0 | **2069**(14.26MPa) | 656 | 379 (2.61 MPa) |
| 6 | **1925**(13.27MPa) | 614 | 368 (2.54 MPa) |
| 48 | **2054**(14.16MPa) | **644** | 436 (3.01 MPa) |
| 100 | **2118**(14.60MPa) | **679** | 486 (3.35 MPa) |

From the above data, thermoplastic elastomers in general are a good alternative to the current PN-3206 silicone material due to their higher starting mechanical properties and their acid stability. Of the materials tested it was shown that SIS14S, SIS22S, SBS, SEBS, PVA, EPDM, and BR have almost no mechanical property loss over time in 12N HCl. This indicates that the materials are extremely acid stable and would likely be entirely unaffected by stomach acid. Many of these materials are also stiffer than the current silicone. This would allow them to be made with a thinner shell which would allow for easier implantation while still having a stronger break force than the current material.

Another parameter that is important to study is the percent loss of mechanical properties for particular materials over time relative to the same type of degradation in the baseline material, PN-3206. That is, all of these materials degrade to a certain degree over time in 38% HCI, but desirable materials degrade less than the baseline. Table VI below gives the results of accelerated testing for the current material, PN-3206, as a percent loss of the various physical properties from the property value prior to acid exposure at times 6 and 100 hours. Table VI also shows the decrease as a percent of initial mechanical properties of various candidate materials after 6 hours and 100 hours in 38% HCl in relation to the baseline material, PN-3206. Lower percentages are better values since they translate into acid stability higher than the baseline, greater percentages are worse values since they translate into acid stability lower than the baseline. In this table, any percent loss of properties at the corresponding time period less than that of the baseline is in bold. This allows easy comparison of the acid stability between the baseline (PN-3206) and the other tested materials.

**Table VI - Physical Property Loss of Current Material, PN-3206, and Various New Materials**

| Test → | % Loss of Ultimate Tensile Strength, UTS | | % Loss of Elongation, Eb | | % Loss of Stiffness, k | |
|---|---|---|---|---|---|---|
| Time (hr) → | | | | | | |
| Material ↓ | 6 hrs | 100 hrs | 6 hrs | 100 hrs | 6 hrs | 100 hrs |
| PN-3206 (baseline) | 50 | 85 | 11 | 62 | 18 | 36 |
| PN-3210 | **43** | **10** | **16** | **80** | **44** | 46 |
| CSM-3930 | **9** | 23 | **-34** | **15** | **33** | -37 |
| LSR-9744 | **-41** | 14 | **-120** | **-6** | **32** | **-133** |
| FS 30 | 94 | 97 | N/A | N/A | N/A | N/A |
| SIS14S | **-55** | **8** | **-33** | **-21** | **8** | **-32** |
| SIS22S | **-5** | **-8** | **10** | **-15** | **-6** | **-5** |
| SBS | **-32** | **-3** | **-5** | **3** | **0** | **1.4** |
| SEBS | **-22** | **0** | **-19** | **6** | **-1** | **4** |
| Elvax | **-1** | **-5** | **6** | **-93** | **27** | **-63** |
| EPDM | **-7** | **0** | **15** | **7** | **8** | **-4** |
| BR | **7** | **6** | **3** | **-2** | **-4** | **-28** |

From these data, it is apparent that the silicones, PN-3210, CSM-3930 and LSR-9744 have a higher acid stability than PN-3206. The other silicone materials tested have a lower acid stability than PN-3206. Both styrene and synthetic rubbers have higher acid stability than PN-3206.

### Thermoplastic Vulcanizates (TPV)

Such materials are not encompassed by the material requirements of the independent claims.

Thermoplastic vulcanizates consist of a crosslinked rubber phase dispersed in a thermoplastic polymer phase. They show excellent mechanical properties such as high extensibility as well as good chemical resistance. Introducing crosslinking into a selected thermoplastic elastomer will result in a thermoplastic vulcanizate with improved mechanical and chemical properties, which are relevant from both manufacturing and prolonged implantation standpoints:
- improved starting UTS
- reduced creep
- increased general chemical resistance, especially to oil and gastric enzymes
- facilitated manufacturing by modulating self-sealing and preserving current processes

Consequently, TPVs will be developed based on selected TPEs. TPVs with different crosslinking levels will be formulated using various peroxide crosslinkers on SIS14S, SIS22S, SBS and SEBS. The resulting materials will be screened for their mechanical and chemical properties.

As mentioned, the graphs in Figures 3-5 show comparisons between the current material (PN-3206) and a number of other particular silicone elastomers, as listed at the bottom of each page. All the silicone rubbers tested except FS 30 were purchased from Nusil Silicone Technology (Carpinteria, CA). FS 30 (ASC part #40130) was purchased from Applied Silicone Corporation (Santa Paula, CA). From all tested silicones, three others are currently used in commercial implantable devices available from Allergan Inc.: MED-4850 in a fill valve for the Orbera® System balloon, PN-6309 for the Orbera® System valve/shell seal and PN-3210 as a barrier layer in the shell of certain breast implants.

The chemical formula for the various silicone elastomers is provided as follows:

Silicone rubbers are polymerized siloxanes. The chemical formula of silicones is [R₂SiO]*ₙ*, where R is an organic group such as:
- phenyl (phenyl silicones):
   PN-3206, 5 wt. % phenyl content
   PN-3210, 15 wt. % phenyl content
- methyl, ethyl or CxHy (methyl silicones):
   MED-4720
   CSM-4755
   MED-6640
   MED-4850
   PN-6309
- a fluorine atom (fluorosilicones):
   CSM-3930, 100% fluorosubsituted
   LSR-9744, 100% fluorosubsituted
   FS 30, 30% fluorosubsituted

Styrene rubbers (pellets) were purchased from Sigma Aldrich (Saint Louis, MO). Styrenes with the following chemical formulas were tested:
- SIS: Polystyrene-block-polyisoprene-block-polystyrene
   SIS14S has a 14 wt. % styrene content, SIS22S has a 22 wt. %styrene content 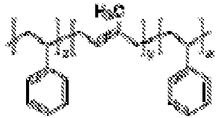
- SEBS: Polystyrene-block-etylene/18utylenes-block-polystyrene, styrene content: 28 wt. % 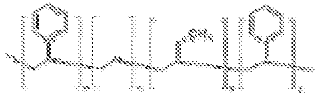
- SBS: Polystyrene-block-polybutadiene-block-polystyrene, styrene content: 30 wt. %

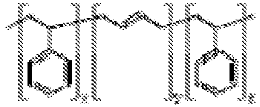

Synthetic rubbers - PEVA (pellets) was purchased from Sigma Aldrich. Synthetic rubbers with the following chemical formulas were tested:
- Poly(ethylene-co-vinyl acetate), vinyl acetate content: 40 wt. %

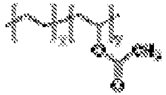

EPDM (sheets) was purchased from McMaster-Carr (Aurora, OH) with the chemical formula: Ethylene propylene diene Monomer (M-class) rubber.

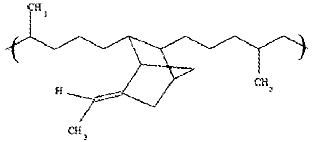

Butyl rubber (gloves used as sheets) was purchased from Ansell (Red Bank, NJ) and has the chemical formula:
- Butyl rubber is a copolymer of 98 wt. % isobutylene with about 2 wt. % isoprene
- The chemical formula of butyl rubber is -(-CH₂-C(CH₃)₂-)ₙ-
- Viton® based fluoroelastomer (dispersion, product #PLV 2069) was purchased from PelSeal Technologies LLC (Newtown, PA)
- Viton® rubbers are generally copolymers of hexafluoropropylene (HFP) and vinylidene fluoride (VDF)
- Viton® is not and will not be approve for use in implantable devices.

Selected new materials with better starting properties and higher acid stability are potential candidates to replace PN-3206, and eventually replace MED-4850 for the fill valve as well.

The graphs in Figures 3-8 display absolute mechanical properties of all tested materials. They show the comparison of starting mechanical properties before accelerated acid testing (t = 0 hours), as well as after 6, 48 and 100 hours in 38% HCl, between the baseline (PN-3206) and the other tested materials.

In general, the materials of interest are equal to or surpass the baseline material. The presence of lower values relative to the baseline in potential candidates mechanical properties can potentially be remediated through chemical modification/crosslinking of these materials. Certain trends are seen:
- The silicone materials tested have in general worse mechanical properties than PN-3206.
- Styrene rubbers have in general better mechanical properties than PN-3206:
   a. SIS14S has equal or better values across the board
   b. SIS22S and SBS have better UTS and elongation but worse stiffness
   c. SEBS has better UTS but worse elongation and stiffness
- Synthetic rubbers have in general better mechanical properties than PN-3206:
   d. PEVA has most values better than 3206
   e. EPDM and Butyl Rubber have better UTS but worse Eb and k
   f. Viton has worse starting mechanical properties but higher UTS and Eb after acid testing

### CONCLUSIONS:

CSM-3930 can be used in place of PN-3206. Nevertheless, CSM-3930 being a RTV (Room Temperature Vulcanization) silicone may result in processability issues due to its propensity to creep over time. Therefore, CSM-3930 is desirably used as a barrier layer along with a different material scaffold. LSR-9744, a similar HTV (High Temperature Vulcanization) material developed by Nusil, has improved processability, but has a lower tear strength than CSM-3930. PN-3210 is believed to be able to form the entire shell, but may also be used merely as a barrier layer along with a different material scaffold.

A number of other ideas have been generated for ways to improve the intragastric balloon and future intragastric devices. These ideas include the use of an antimicrobial coating, responsive materials, cycling materials, new fill materials, and adding self sealing properties of the balloon. Below is a brief description of the ideas with main focus headings and application subheadings.

### Anti-microbial Coatings

Because of the length of time that the proposed intragastric balloons will reside in the stomach, it is important to ensure against bacterial infections and the like. Consequently, an anti-microbial coating is desirable that directly reduces and inhibits bacterial colonization of the device and used clinically, and can potentially prolong the clinical life of the device by inhibiting/limiting bacterial colonization and infection/necrosis. Is suggested that bacterial adherence, colonization, and subsequent presence of necrotic tissue plays a role in the degradation of the balloon material. As a consequence, the anti-microbial coating thus prevents localized stress, damage and degradation of material properties of the raw material used to manufacture the device

The use of some current intragastric balloon devices is limited to six months *in vivo* mainly due to deterioration of physical properties of the silicon used in the shell that may potentially result in failure (cracks, holes, tears). It is believed that in the absence of failures due to bacterial interactions, the physical properties of the existing balloon materials are sufficient to last greater than six months. In fact, bacterial interaction are believed to have a major role in creating localized zones of weaker material due to a mechanism known as "environmental stress cracking." See for example, "The most common postoperative complication of indwelling devices and implants is infection," (Medical Disability Advisor, 2009).

An outer surface of an intragastric balloon that is less resistant to bacterial colonization may be able to reduce degradation of the balloon material beyond what is seen in currently use devices. Any of the intragastric balloons described herein may therefore include an additional outer anti-microbial coating or layer. For example, a silicone dispersion such as PM-3206 or PM-3210 may be used to create underlying layers of the balloon, while the outer layers are modified to provide the antimicrobial characteristic. The surface modification could be in the form of a coating or layer containing a known anti-microbial agents such as silver (or pharmaceutical such as rifampicin, vycomicin, etc.). The anti-microbial coating or layer is desirably non-adhesive so as to resist attachment of bacteria. This could be achieved using surface coatings such as PTFE (Teflon), Parylene, etc.

In one method, the outer surface of an intragastric balloon may be covered by a silicone layer mixed with an antimicrobial agent (e.g., silver to produce silver ions). The preferred solution is to coat additional layers on the balloon surface using a silicone dispersion containing the anti-microbial agent such that the surface is completely covered by this layer in its clinically relevant expanded configuration. Another method is modification of the balloon surface by physical vapor deposition of thin film coatings. A still further method includes plasma coating of anti-microbial agents or non-adhesive surfaces such as PTFE. One advantage of these methodologies is that the additional coating is the last step in the balloon assembly and would not significantly change the existing manufacturing steps. Alternatively, the antimicrobial agent may be incorporated into the raw material used to mold the balloon shell.

The material used for the outer antimicrobial coating or layer should be biocompatible and must be able to coat the balloon surface relatively uniformly. The coating layer must stay attached to the substrate materials, and additionally must possess acid resistance, abrasion resistance, and compatibility with device manufacturing, sterilization, storage (over shelf life) and shipping environments.

### Responsive Materials

For instance, some polymeric coatings that are pH responsive may expand to cause a softer interaction between the strut of the structure and the stomach wall. Other materials have acid backbone functionalities that contract in a low pH. Still further, intragastric devices may include a base material made using two crosslinkers, one permanent and another temporary that breaks down in acid. Alternatively, a material that expands in response to an acidic environment and results in a stiffer material may be desirable. A material may be used in the leak valve that swells or contracts in the presence of acid to reduce the likelihood of leaks.

### Application: Responsive Shell

Changes in Force due to pH and or changes in Volume due to pH. Polymeric coatings that are pH responsive that can expand to cause a softer interaction between the strut of the structure and the stomach wall. Some examples of materials are Nitinol (base structure) coating materials: lightly crosslinked chitosan or poly-amine or any materials that have positive charged functional groups on the background or the pendent. This affords up to 1000% expansion in wet acidic environments.

Some examples of materials which shrink in acid are lightly crosslinked PVA fully deacetylated to somewhat deacetylated, lightly crosslinked polyacrylic acid or any materials with a side group alcohol or carboxylic acid group, either directly on the backbone or on a pendant, alternatively some materials which can expand in acid are lightly crosslinked polyethyleneamine, chitosan deacetylated from between 60 and 100%.

### Application: Slow Expansion Device

A device with the base material made using two cross-linkers. One permanent and the other temporary that breaks down in acid.

This is more of a single material (may be more than 1) which has 2 types of x-linkers, one which is permanent and not degraded in the environment, and the second which is slowly degraded in the environment, an example may be a silicone chain that is slowly cleaved by acid, or a polycarbonate, or polyurethane, some polyesters are acceptable as well. The material should be swellable in the environment and will expand as the second crosslinker is degraded and allows the chains to unwind. The first crosslinker will keep the material from fully dissolving in the medium, since materials which typically swell in a solvent will dissolve unless something like a permanent x-linker binds them together.

### Application: Composite Wall

A material that expands in response to an acidic environment results in a stiffer material. A responsive increase in stiffness to acid. It is advantageous to have the balloon wall as soft as possible for packaging and pre-insertion, however there are some advantages to increasing the stiffness of the balloon wall once it has been implanted. This can be achieved by dispersing particulates of material which is hydrophilic into a layer of the wall (or throughout the entire thickness of the BIB wall). Once the BIB wall is exposed to a wet environment, the hydrophilic material will uptake water, and swell causing stresses to be formed within the BIB wall. This can significantly stiffen the BIB wall. Some hydrophilic materials can be generally hydrophilic polymer particles or particles of gel. Some examples include powdered polyethylene glycol, or lightly crosslinked polyacrylic acid, polyvinyl alcohol, etc.

Example 1: A silicone shell is made by dipping a mandrel into 25% weight by weight MED-6640 HTV silicone in Xylene. The layer is allowed to dry at room temperature and a second and third coat are added allowing the shell to dry between coats. The silicone remains tacky. The higher phenyl content results in an improved acid stability because it has not yet been vulcanized. The shell is then dipped into a fluidized bed containing sodium polyacrylate particles. The particles coat and cover the entire shell. The samples is then coated twice more with 20% w/w MED-6640 in Xylene. The shell is then vulcanized at 126°C for 1 hour. The shell is removed from the mandrel and is finished. When the shell comes into contact with water the sodium polyacrylate particles gradually swell to create a self sealing force that is resistant to punctures and pin hole leaks.

Example 2: A composite material shell of an intragastric balloon is made by dipping a mandrel into 25% weight by weight MED-6600 HTV silicone in Xylene. The layer is cured at 140°C for 20 minutes. The shell is then dipped into a 5% w/w PVA solution in water. The layer is allowed to dry and then is dipped into a 10% borax solution in water. After 1 minutes the mandrel is removed and washed in DI water. The shell is dried at 45°C for 15 minutes and then 2 more layers of MED-6600 HTV silicone are applied to the shell. The final result is a composite shell with a thin layer (approximately 10% of the thickness of the 1mm shell) of lightly crosslinked polyvinyl alcohol. When the intragastric balloon is placed into the stomach the PVA swells causing stress to be formed within the shell wall significantly stiffening it.

### Application: Responsive Valve

The materials for this application are the same as the materials listed in the responsive shell application. The purpose and advantage of this application is that it will reduce the ability for the valve to leak which should reduce adverse events.

The use of a material in the leak valve that swells when contacted by acid or liquid in order to have it compress the slit in the valve when acid migrates into the balloon thus reducing the chance for the valve to leak. A part or all of the valve may be swellable, or alternatively contractible in order to close all or a part of the valve after the balloon has been implanted. The swellability can be impacted by increase in temperature, decrease in pH or contact with fluid (saline).

Example 1: A groove in the valve where the slit is placed and then to put a circular band of material that fits perfectly into the grove. The material would contract when exposed to acid caused by acid diffusion into the balloon. This would cause pressure to be applied to the valve slit, forcing it closed.

Example 2: The valve entrance could be made with a pH sensitive material that will constrict when it comes into contact with acid. This would cause a force to be applied to the valve pulling on the valve stem and causing it to seal better.

Example 3: The entire inside of the valve could be coated with a polymer that expands in contact with acid. This could cause the inside of the valve to swell up and close once the device had been implanted.

Example 4: The outside of the valve could be made of a polymer that expands when in contact with acid. This would cause a force to be applied to the valve applying pressure to keep the slit closed.

Example 5: The valve slit could be lined with a material that expands in acid in order to increase the sealing ability of the valve.

Example 6: Any of these examples can be used together to improve the sealing ability of the valve. For instance example 1 and example 6 could be combined to create an area were the material is swelling on the inside of the valve while a band is applying a constrictive force from outside the valve.

### Cycling Materials

One option is an intragastric device that expands and contracts due to a stimulus, occasionally or regularly throughout the day. The main purpose for the use of cycling materials is to prevent the stomach from stretching. It has been shown that the stomach becomes acclimated over time to a continual stress. By having the BIB expand and contract throughout the day this can be averted resulting in a stronger therapeutic effect. For instance, if the BIB is actively contracted by the pH increasing in the stomach then before sleeping the patient could take antacids that will significantly increase stomach pH causing the device to contract. This would allow there stomach to shrink back to its normal size so that when the device expands again it has a stronger effect.

### Actively Cycling Materials

In an actively cycling material, an expansion in volume may be induced by mechanical agitation, cause by ingestion of food. The material can expand and occupy more space, after the person feels full, and food intake - mechanical agitation stops, the material may return to its original unexpanded form in order to allow the stomach walls to relax. Alternatively, the material may expand or contract due to changes in pH of the stomach, which are typically associated with hunger or food intake. Materials which are sensitive to small changes in pH must have a specific balance of basic and/or acidic functionalities on the backbone or pendant chains.

### Passively Cycling Materials

Passively cycling materials may expand and contract regularly throughout the day. Preferably the cycle coincides with contracture at night, when the patient is sleeping so the stomach isn't stretched and can return to its normal geometry, while expansion during the day, so that the majority of the stomach volume is occupied. Alternatively the day cycle can have several stretch and contracture points which are set to coincide with typical feeding time for the patient. Essentially any polymer that has pendant groups of carboxylic acid, amine, alcohol, or other potentially charged moieties. The idea is that at different pHs the material will take on different charges based on its pKa. Consequently, one can control at which pH a material becomes crosslinked and how fast the transition occurs. This allows materials to be tailored so that at certain pH's they are hydrophylic and swell, increasing in size, while at other pH's they are hydrophobic and shrink. This allows for controlled cycling of the expansion and contraction of a balloon.

Specific Examples: Materials that can do this are polyethyleneimine, Chitosan, Polyhydroxyalkanoate, and Carboxymethyl Cellulose.

### Materials swelling in oil (explain):

Can be used as responsive shell or patch material, for volume modulation or as self-sealing material; for example, EPDM.

### Fill Materials

We have been able to show in the lab that different fill materials can affect the ability of microbial infection to occur inside the balloon. As the explanted BIBs have shown, microbial infection can lead to catastrophic failure of the device. Since doctors currently add their own filler, deciding on how much methylene blue to add and what type of buffer to use, it would be best to standardize the fill material. The additives to the fill material could also serve to better negate acid that migrates into the balloon reducing acid degradation from the inside out. It could also be bacteriostatic and hinder bacteria from colonizing inside the device which could potentially reduce the risk of device failure.

### Standardized fill solution

One configuration is a particular concentration of salts, acidic composition or a buffer, methylene blue, etc that is optimized for the material. May have bacteriostatic or antibiotic properties, which may or may not diffuse out through the wall of the BIB

Examples: A specific concentration of methylene blue and saline; or sodium citrate may be used both as pH buffer and a potential bacteriostatic.

### Different Fill Material

A material which when degraded by acid acts as a buffer.

For instance, the fill material may be lighter. This may be a closed cell foam, or fragments of a closed cell foam which are suspended in a carrier fluid

Another potential filler material modifies the properties of the BIB. For example, a locking solution type material, which does one or more of: limits diffusion, enhances stiffness of the balloon, reduces vibration frequency, contains an antibiotic solution to prevent the inside of the BIB from getting infected, or slowly elutes out the antibiotic solution through the BIB wall to prevent the BIB wall from getting colonized by bacteria, the material may perhaps turn into a viscous fluid or gel at body temperature, some such materials may include reversibly thermogelling materials or materials which are shear thinning

Specific Examples: Reversibly thermogelling materials such as solutions of methyl cellulose, pluronic; or Materials which are shear thinning, such as guar gum, xanthan gum, hyaluronic acid

Another filler material may continuously expand. Some available intragastric devices allow for a controlled filling of the balloon with a saline solution, to promote continuous weight loss. An optional filler material may be a solution which through osmotic mechanisms promotes diffusion of liquid from the outside into the balloon to slowly increase in volume. The diffusion mechanisms may be controlled by the type of ion that is creating the osmotic force, the shell material which is limiting diffusion and the viscosity of the solution inside the BIB. For instance a more viscous solution will limit diffusion and increase in volume slower, a gel will have a maximum swell volume, etc.

Specific Examples: a Biocompatible Gel - PVA Gel; or Materials that swell in saline, such as sodium polyacrylate and crosslinked poly(isobutylene-co-maleic acid) sodium salt

### Basic or Buffered Fill Material

The filler material may be buffered against acid degradation from the inside out. It has been shown that acid is able to penetrate through the BIB shell and equilibrate with the solution that is used to fill the BIB. This causes BIB degradation to occur from both the outside and the inside.

### Specific Examples: sodium citrate as a pH buffer.

### Self Sealing Balloon

A balloon that has self sealing characteristics which allow it to resist leaking even after the shell has a pinhole defect.

Some tests have shown that the BIB might rupture due to pin hole defects in the shell possibly caused by increased levels of silica in the shell at a specific point, poor polymerization of the silicone, a high concentration of cyclics used in silicone polymerization, or an external force. The addition of a sealing layer would allow the BIB to seal such pin hole defects instead of leaking and or deflating.

### Example: Double Shell with Gel

A double lumen silicone shell is made with a layer of fully cured silicone gel in the center. The total shell thickness is approximately 1.5mm. The silicone gel can be given additives so that it swells in response to water, further increasing its self sealing ability.

### Example: PVA inner lining

A silicone shell is dipped and cured. A thin 0.5mm layer of PVA is added to the shell and crosslinked using 10% Borax in water. The shell is then flipped inside out. When the device is filled the saline causes the PVA to swell which creates the self sealing characteristics.

### Example: Double Shell with PVA

A double lumen silicone shell is made with a layer of crosslinked PVA in the center. The total shell thickness is approximately 1.0mm. The PVA is crosslinked so that it will swell but not dissolve in the presence of water. When a puncture is made in the shell, the PVA swells to stop the balloon from leaking from that spot.

### Example: Sodium Polyacrylate particles

A silicone shell is made by dipping a mandrel into 20% weight by weight MED-6640 HTV silicone in Xylene. The layer is allowed to dry at room temperature and a second and third coat are added allowing the shell to dry between coats. The silicone remains tacky because it has not yet been vulcanized. The shell is then dipped into a fluidized bed containing sodium polyacrylate particles. The particles coat and cover the entire shell. The samples is then coated twice more with 20% w/w MED-6640 in Xylene. The shell is then vulcanized at 126°C for 1 hour. The shell is removed from the mandrel and is finished. When the shell comes into contact with water the sodium polyacrylate particles gradually swell to create a self sealing force that is resistant to punctures and pin hole leaks.

## Claims

1. An intragastric balloon for the treatment of obesity, comprising:
(a) a shell having a wall enclosing an inner volume, and;
(b) a fluid fill valve secured in the shell wall,
wherein a barrier layer of the shell wall is made of a 100% fluorinated silicone.

2. The intragastric balloon of claim 1, wherein the shell wall includes an outer barrier layer, as the barrier layer, of the 100% fluorinated silicone and an inner scaffold layer of a different material.

3. The intragastric balloon of claim 2, wherein the outer barrier layer is between about 5- 25% of the total shell wall thickness, or
wherein the outer barrier layer is about 20% of the total shell wall thickness.

4. The intragastric balloon of claim 2, wherein the inner scaffold layer is a 5% phenyl- substituted high-temperature vulcanization (HTV) silicone, or
wherein the inner scaffold layer is a methyl silicone, or
wherein the inner scaffold layer is a 100% fluorinated high-temperature vulcanization (HTV) silicone.

5. The intragastric balloon of claim 2, further including a sealing layer in addition to the outer barrier layer and inner scaffold layer to provide a self-sealing shell in case of rupture.

6. The intragastric balloon of claim 1, further comprising an outer coating having an antimicrobial agent therein coated on the outer barrier layer.

7. An intragastric balloon for the treatment of obesity, comprising:
(a) a shell having a wall enclosing an inner volume, and;
(b) a fluid fill valve secured in the shell wall,
wherein the shell wall, or a barrier layer thereof, is made of a 15-30% phenyl-substituted high-temperature vulcanization (HTV) silicone.

8. The intragastric balloon of claim 7, wherein the shell wall is made of a 15% phenyl- substituted high-temperature vulcanization (HTV) silicone.

9. The intragastric balloon of claim 7, wherein the shell wall includes an outer barrier layer of the 15-30% phenyl-substituted high-temperature vulcanization (HTV) silicone and an inner scaffold layer of a different material.

10. The intragastric balloon of claim 9, wherein the outer barrier layer is between about 5- 25% of the total shell wall thickness, or
wherein the outer barrier layer is about 20% of the total shell wall thickness.

11. The intragastric balloon of claim 8, wherein the inner scaffold layer is a 5% phenyl-substituted high-temperature vulcanization (HTV) silicone.

12. The intragastric balloon of claim 9, wherein the inner scaffold layer is a methyl silicone.

13. The intragastric balloon of claim 9, wherein the inner scaffold layer is a 100% fluorinated high-temperature vulcanization (HTV) silicone.

14. The intragastric balloon of claim 9, further including a sealing layer in addition to the outer barrier layer and inner scaffold layer to provide a self-sealing shell in case of rupture.

15. The intragastric balloon of claim 7, further comprising an outer coating having an antimicrobial agent therein coated on the outer barrier layer.

## Patentansprüche

1. Intragastrischer Ballon zur Behandlung von Fettleibigkeit, umfassend:
(a) eine Hülle mit einer Wand, die ein Innenvolumen umschließt, und;
(b) ein Fluidfüllventil, befestigt in der Hüllenwand,
wobei eine Sperrschicht der Hüllenwand aus einem 100 % fluorierten Silikon hergestellt ist.

2. Intragastrischer Ballon nach Anspruch 1, wobei die Hüllenwand als Sperrschicht eine äußere Sperrschicht aus dem 100 % fluorierten Silikon und eine innere Gerüstschicht aus einem anderen Material enthält.

3. Intragastrischer Ballon nach Anspruch 2, wobei die äußere Sperrschicht zwischen etwa 5 - 25 % der gesamten Hüllenwanddicke ist, oder
wobei die äußere Sperrschicht etwa 20 % der gesamten Hüllenwanddicke ist.

4. Intragastrischer Ballon nach Anspruch 2, wobei die innere Gerüstschicht ein 5 % Phenyl-substituiertes Hochtemperatur-Vulkanisations (HTV)-Silikon ist, oder
wobei die innere Gerüstschicht ein Methylsilikon ist, oder
wobei die innere Gerüstschicht ein 100 % fluoriertes Hochtemperatur-Vulkanisations (HTV)-Silikon ist.

5. Intragastrischer Ballon nach Anspruch 2, weiter enthaltend eine Abdichtungsschicht zusätzlich zu der äußeren Sperrschicht und inneren Gerüstschicht zum Bereitstellen einer selbstabdichtenden Hülle im Fall von Reißen.

6. Intragastrischer Ballon nach Anspruch 1, weiter umfassend eine äußere Beschichtung mit einem antimikrobiellen Mittel darin, beschichtet auf die äußere Sperrschicht.

7. Intragastrischer Ballon zur Behandlung von Fettleibigkeit, umfassend:
(a) eine Hülle mit einer Wand, die ein Innenvolumen umschließt, und;
(b) ein Fluidfüllventil, befestigt in der Hüllenwand,
wobei die Hüllenwand oder eine Sperrschicht davon aus einem 15-30 % Phenyl-substituierten Hochtemperatur-Vulkanisations (HTV)-Silikon hergestellt ist.

8. Intragastrischer Ballon nach Anspruch 7, wobei die Hüllenwand aus einem 15 % Phenyl-substituierten Hochtemperatur-Vulkanisations (HTV)-Silikon hergestellt ist.

9. Intragastrischer Ballon nach Anspruch 7, wobei die Hüllenwand eine äußere Sperrschicht aus dem 15-30 % Phenyl-substituierten Hochtemperatur-Vulkanisations (HTV)-Silikon und eine innere Gerüstschicht aus einem anderen Material enthält.

10. Intragastrischer Ballon nach Anspruch 9, wobei die äußere Sperrschicht zwischen etwa 5- 25 % der gesamten Hüllenwanddicke ist, oder
wobei die äußere Sperrschicht etwa 20 % der gesamten Hüllenwanddicke ist.

11. Intragastrischer Ballon nach Anspruch 8, wobei die innere Gerüstschicht ein 5 % Phenyl-substituiertes Hochtemperatur-Vulkanisations (HTV)-Silikon ist.

12. Intragastrischer Ballon nach Anspruch 9, wobei die innere Gerüstschicht ein Methylsilikon ist.

13. Intragastrischer Ballon nach Anspruch 9, wobei die innere Gerüstschicht ein 100 % fluoriertes Hochtemperatur-Vulkanisations (HTV)-Silikon ist.

14. Intragastrischer Ballon nach Anspruch 9, weiter enthaltend eine Abdichtungsschicht zusätzlich zu der äußeren Sperrschicht und inneren Gerüstschicht zum Bereitstellen einer selbstabdichtenden Hülle im Fall von Reißen.

15. Intragastrischer Ballon nach Anspruch 7, weiter umfassend eine äußere Beschichtung mit einem antimikrobiellen Mittel darin, beschichtet auf die äußere Sperrschicht.

## Revendications

1. Ballon intragastrique pour le traitement de l'obésité, comprenant :
(a) une enveloppe ayant une paroi renfermant un volume interne, et ;
(b) une valve de remplissage de fluide fixée dans la paroi d'enveloppe,
dans lequel une couche barrière de la paroi d'enveloppe est faite d'une silicone 100 % fluorée.

2. Ballon intragastrique selon la revendication 1, dans lequel la paroi d'enveloppe inclut une couche barrière externe, en tant que couche barrière, de la silicone 100 % fluorée et une couche de structure interne d'un matériau différent.

3. Ballon intragastrique selon la revendication 2, dans lequel la couche barrière externe représente d'environ 5 à 25 % de l'épaisseur de paroi d'enveloppe totale, ou
dans lequel la couche barrière externe représente environ 20 % de l'épaisseur paroi d'enveloppe totale.

4. Ballon intragastrique selon la revendication 2, dans lequel la couche de structure interne est une silicone de vulcanisation haute température (HTV) substituée à 5% par du phényle, ou
dans lequel la couche de structure interne est une silicone méthylique, ou
dans lequel la couche de structure interne est une silicone de vulcanisation haute température (HTV) 100 % fluorée.

5. Ballon intragastrique selon la revendication 2, incluant en outre une couche d'étanchéité en plus de la couche barrière externe et de la couche de structure interne pour fournir une enveloppe auto-étanche en cas de rupture.

6. Ballon intragastrique selon la revendication 1, comprenant en outre un revêtement externe ayant un agent antimicrobien dans celui-ci revêtant la couche barrière externe.

7. Ballon intragastrique pour le traitement de l'obésité, comprenant :
(a) une enveloppe ayant une paroi renfermant un volume interne, et ;
(b) une valve de remplissage de fluide fixée dans la paroi d'enveloppe,
dans lequel la paroi d'enveloppe, ou une couche barrière de celle-ci, est faite d'une silicone de vulcanisation haute température (HTV) substituée à 15 à 30 % par du phényle.

8. Ballon intragastrique selon la revendication 7, dans lequel la paroi d'enveloppe est faite d'une silicone de vulcanisation haute température (HTV) substituée à 15% par du phényle.

9. Ballon intragastrique selon la revendication 7, dans lequel la paroi d'enveloppe inclut une couche barrière externe de la silicone de vulcanisation haute température (HTV) substituée à 15 à 30 % par du phényle et une couche de structure interne d'un matériau différent.

10. Ballon intragastrique selon la revendication 9, dans lequel la couche barrière externe représente d'environ 5 à 25 % de l'épaisseur de paroi d'enveloppe totale, ou
dans lequel la couche barrière externe représente environ 20 % de l'épaisseur paroi d'enveloppe totale.

11. Ballon intragastrique selon la revendication 8, dans lequel la couche de structure interne est une silicone de vulcanisation haute température (HTV) substituée à 5 % par du phényle.

12. Ballon intragastrique selon la revendication 9, dans lequel la couche de structure interne est une silicone méthylique.

13. Ballon intragastrique selon la revendication 9, dans lequel la couche de structure interne est une silicone de vulcanisation haute température (HTV) 100 % fluorée.

14. Ballon intragastrique selon la revendication 9, incluant en outre une couche d'étanchéité en plus de la couche barrière externe et de la couche de structure interne pour fournir une enveloppe auto-étanche en cas de rupture.

15. Ballon intragastrique selon la revendication 7, comprenant en outre un revêtement externe ayant un agent antimicrobien dans celui-ci revêtant la couche barrière externe.
